# EUROPEAN PATENT APPLICATION

(11) **EP 1 911 829 A1**
(43) Date of publication of application: **16.04.2008**
(21) Application number: 06255207.0
(22) Date of filing: 10.10.2006
(51) Int. Cl.: C10G 21/27, C07C 211/63, C07D 233/54, C07D 521/00

(54) **Process for deacidifying crude oil**

(71) Applicant: The Queen's University of Belfast, Belfast BT7 1NN, Northern Ireland (GB)
(72) Inventor: The designation of the inventor has not yet been filed
(74) Representative: Hamer, Christopher K.

(57) **Abstract**

A process for deacidifying a crude oil and/or crude oil distillate containing organic acids comprising the steps of:
(a) contacting the crude oil and/or crude oil distillate containing organic acids with a basic ionic liquid having a melting point of below 150°C, and extracting at least a portion of the organic acids into the basic ionic liquid as an extract phase; and
(b) separating a crude oil and/or crude oil distillate phase which is reduced in acidity from the basic ionic liquid extract phase.

## Description

This invention relates to a process for deacidification of crude oil and/or a crude oil distillate.

Crude oil and distilled fractions thereof may contain organic acid impurities such as naphthenic acids. Typical Total Acid Number (TAN) values, as measured by ASTM Method D0664, for acidic crude oils are in the range of 0.5 to 4 mg KOH/g while acidic distilled fractions such as kerosene may have TAN values of, for example, 0.5 to 8 mg KOH/g. These organic acid impurities can cause corrosion problems, particularly in refinery operations where temperatures of 200°C and above are encountered. For this reason, it is desirable to reduce the acidity of crude oils and/or crude oil distillates, for example, by reducing the amount of organic acids, such as naphthenic acids present in the crude oil and/or crude oil distillates.

Various methods for deacidifying crude oil and/or crude oil distillates are known. In a conventional deacidification process, an alkali such as aqueous sodium hydroxide or aqueous potassium hydroxide is contacted with the oil to neutralize any acid present. The reaction produces an aqueous phase comprising water, and alkali metal naphthenate. This aqueous phase has to be removed from the deacidified oil, before the oil can be used or sold. According to US 4 199 440, a problem arises in that alkali metal naphthenates are chemically similar to soap, and tend to emulsify hydrocarbon and aqueous phases. This emulsion interferes with the efficient separation of the deacidified oil and aqueous phase.

A further example of a crude oil deacidification process is described in PCT patent application WO 00/46322. In this application, a crude oil is contacted with a polar solvent (for example, methanol), such that at least part of the organic acid present in the oil is extracted into the solvent as an extract phase. The extract phase is then separated from the oil. However, a problem with this process is that certain of the acid impurities are not extractable into the solvent. A further problem is that the organic acid partitions between the oil and the extract phase such that high amounts of polar solvent and repeated extractions are required to reduce the acid content of the oil to an acceptable level. This has a further disadvantage that large volumes of polar solvent need to be regenerated for recycling to the extraction stage.

It has now been found that crude oils or crude oil distillates may be deacidified by extracting the contaminating organic acids into a basic ionic liquid. The basic ionic liquid is highly efficient in extracting the organic acids and may be readily separated from the crude oil and/or crude oil distillates.

Thus, the present invention relates to a process for deacidifying a crude oil and/or crude oil distillate containing organic acids comprising the steps of:
(a) contacting the crude oil and/or crude oil distillate containing organic acids with a basic ionic liquid and extracting at least a portion of the organic acids into the basic ionic liquid as an extract phase; and
(b) separating a crude oil and/or crude oil distillate phase which is reduced in acidity from the basic ionic liquid extract phase.

Examples of organic acids that may be present in the crude oil and/or crude oil distillate include phenolic acids, sulfur-containing acids, and most commonly, naphthenic acids. The person skilled in the art would understand that naphthenic acids are predominately monocarboxylic acids having saturated cyclic structures but also include long-chain aliphatic monocarboxylic acids. By extracting the organic acids into the basic ionic liquid, the TAN value of the oil may be reduced to 0.9 mg KOH/g and below, preferably, 0.5 mg KOH/g and below, and most preferably, 0.3 mg KOH/g and below.

Examples of suitable crude oil distillates which may be deacidified using the process of the present invention include liquefied petroleum gas, gasoline, gas oil, diesel, jet fuel, kerosene, home heating oil and mixtures thereof. Hereinafter crude oil and/or crude oil distillate is referred to as "oil".

The basic ionic liquid may be selected from any liquid salt having the general formula C⁺ A⁻; where C⁺ is a cation having a basic moiety and/or A⁻ is an anion having a basic moiety and the anion, A⁻, forms a liquid salt with the cation, C⁺, at a temperature below 150°C, preferably below 100°C. Thus, the salt is in a liquid state at the temperature of contacting step (a) and separation step (b).

By "basic moiety" is meant that the moiety acts as a base i.e. has an available lone pair of electrons.

Where the cation, C⁺, has a basic moiety, it is preferably represented by the formula:

[Cat⁺-(Z-Bas)ₙ]

wherein:
Cat⁺ is a positively charged moiety:
Bas is a basic moiety;
Z is a covalent bond joining Cat⁺ and Bas, or is a divalent linking group; and
n is an integer of from 1 to 3, preferably 1.

Suitably, Bas comprises at least one basic nitrogen, phosphorus, sulfur, or oxygen atom, preferably, at least one basic nitrogen atom. For example, Bas may comprise a heterocyclic ring system containing a basic nitrogen atom, for example a pyrrolidine or a piperidine ring. Preferably, Bas is selected from -N(R¹)(R²), -P(R¹)(R²), -SR³, -OR³. Suitably, R¹ and R² are independently selected from hydrogen, linear or branched alkyl, cycloalkyl, aryl and substituted aryl, or, in the case of an -N(R¹)(R²) group, R¹ and R² together with the interjacent nitrogen atom form part of a heterocyclic ring. Suitably, R³ is selected from linear or branched alkyl, cycloalkyl, aryl and substituted aryl. Preferably, R¹, R² and R³ are selected from methyl, ethyl, n-propyl, isopropyl, n-butyl, iso-butyl, tert-butyl, n-pentyl, n-hexyl, cyclohexyl, benzyl and phenyl, or, in the case of an -N(R¹)(R²) group, R¹ and R² together represent a tetramethylene or pentamethylene group optionally substituted by one or more C₁₋₄ alkyl groups. Preferably, the basic moiety is a "hindered basic group" i.e. is a functional group that acts as a base and, owing to steric hindrance, does not chemically bond to any of the components of the oil (other of course than by accepting a proton in the usual reaction of a Brønsted acid with a Brønsted base). Suitable hindered basic groups include -N(CH(CH₃)₂)₂ and -N(C(CH₃)₃)₂. Preferably, the hindered basic group has a lower nucleophilicity (or greater steric hindrance) than -N(C₂H₅)₃.

Preferably, Z is a divalent organic radical having from 1 to 18 carbon atoms, preferably 1 to 8 carbon atoms, more preferably, 2 to 6 carbon atoms. The divalent organic radical, Z, may be branched or unbranched. The divalent organic radical, Z, may be substituted or unsubstituted. Preferably, the valence bonds are on different carbon atoms of the divalent organic radical, Z. Suitably, the divalent organic radical, Z, is a divalent aliphatic radical (for example, alkylene, alkenylene, cycloalkylene, oxyalkylene, oxyalkyleneoxy, alkyleneoxyalkylene or a polyoxyalkylene) or is a divalent aromatic radical (for example, arylene, alkylenearylene or alkylenearylenealkylene). Preferably, Z is:
(a) a divalent alkylene radical selected from:
   -(CH₂-CH₂)-, (CH₂-CH₂-CH₂)-, -(CH₂-CH₂-CH₂-CH₂)-, -(CH₂-CH₂-CH₂-CH₂-CH₂)-, -
   (CH₂-CH₂-CH₂-CH₂-CH₂-CH₂)-, -(CH₂-CH(CH₃))-, and -(CH₂-CH(CH₃)-CH₂-CH(CH₃))-;
(b) a divalent alkyleneoxyalkylene radical selected from:
   - (CH₂₋CH₂-O-CH₂-CH₂)-, -(CH₂-CH₂-O-CH₂-CH₂-CH₂)-, and -(CH₂₋CH(CH₃)-OCH₂-CH(CH₃))-;
(c) a divalent polyoxyethylene radical selected from:
   - (CH₂CH₂O)ₙ- where n is an integer in the range 1 to 9 or -(CH₂CH(CH₃)O)ₘ- where m is an integer in the range 1 to 6; and
(d) a divalent alkylenearylene or an alkylenearylenealkylene radical selected from:
   - (CH₂-C₆H₄)-, and -(CH₂-C₆H₄-CH₂)-.

The Cat⁺ moiety may comprise a heterocyclic ring structure selected from imidazolium, pyridinium, pyrazolium, thiazolium, isothiazolinium, azathiozolium, oxothiazolium, oxazinium, oxazolium, oxaborolium, dithiozolium, trizdium, selenozolium, oxaphopholium, pyrollium, borolium, furanium, thiophenium, phospholium, pentazolium, indolium, indolinium, oxazolium, isooxazolium, isotriazolium, tetrazolium, benzofuranium, diborzofuranium, benzothiophenium, dibenzothiophenium, thiadiazolium, pyrimidinium, pyrazinium, pyridazinium, piperazinium, piperidinium, morpholenium, pyranium, annolinium, phthalzinium, quinazolinium, quinaxalinium, quinolinium, isoquinolinium, thazinium, oxazinium and azaannulenium.

Examples of Cat⁺-Z-Bas where Cat⁺ is a heterocyclic ring structure include: 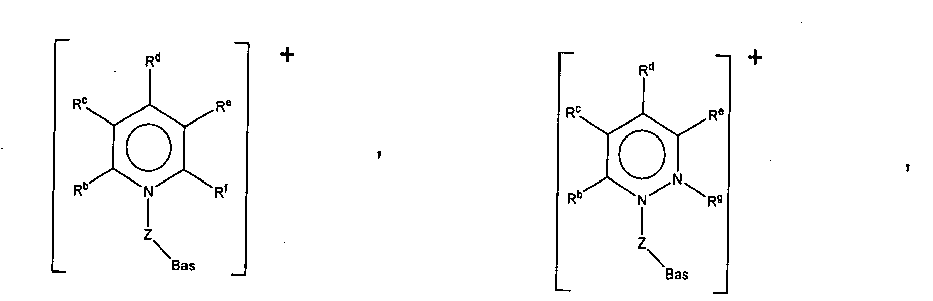 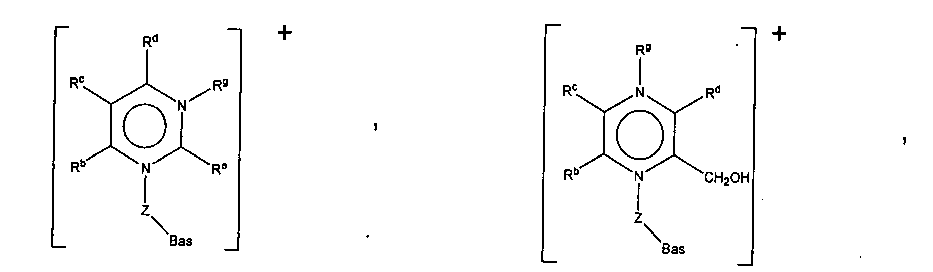 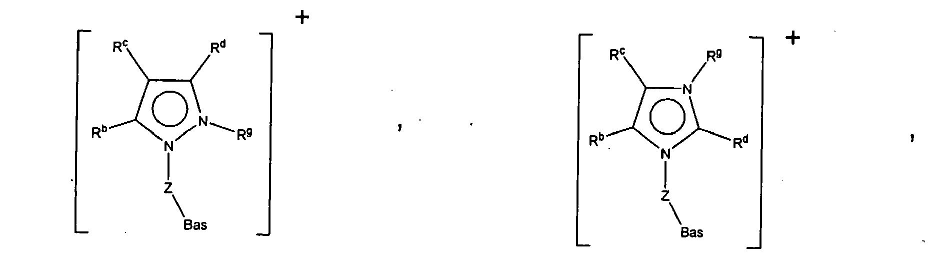 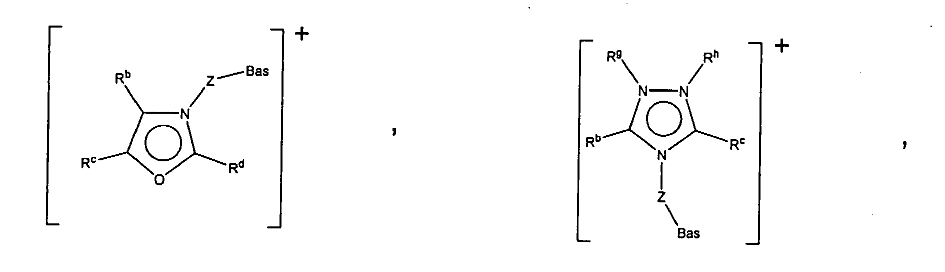 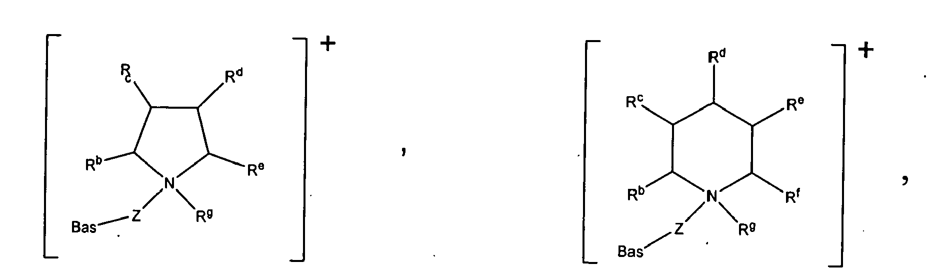 and 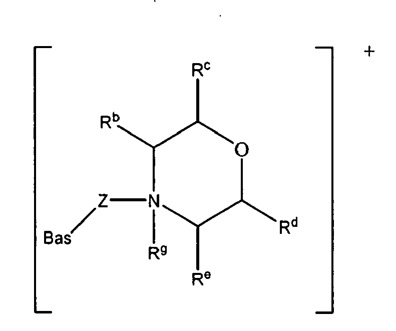 wherein: Bas and Z are as defined above; and R^{b}, R^{c}, R^{d}, R^{e}, R^{f} R^{g} and R^{h} are independently selected from hydrogen, a C₁ to C₄₀, straight chain or branched alkyl group, a C₃ to C₈ cycloalkyl group, or a C₆ to C₁₀ aryl group, wherein said alkyl, cycloalkyl or aryl groups are unsubstituted or may be substituted by one to three groups selected from: C₁ to C₆ alkoxy, C₆ to C₁₀ aryl, CN, OH, NO₂, C₇ to C₃₀ aralkyl and C₇ to C₃₀ alkaryl, or any two of R^{b}, R^{c}, R^{d}, R^{e} and R^{f} attached to adjacent carbon atoms on the ring structure form a methylene chain -(CH₂)ₚ- wherein p is an integer from 3 to 5.

Preferred Cat⁺-Z-Bas where Cat⁺ is a heterocyclic ring structure include: 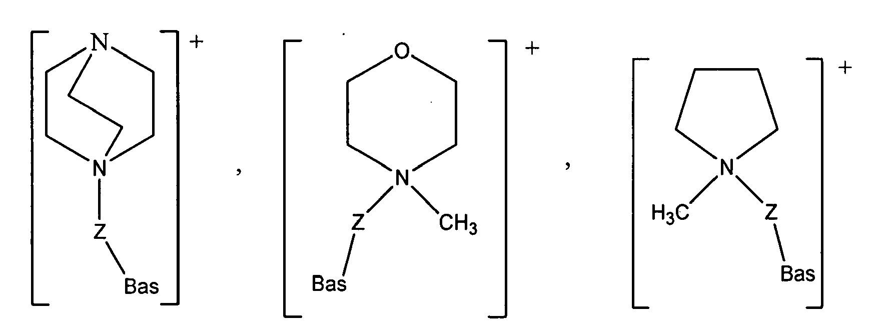 and 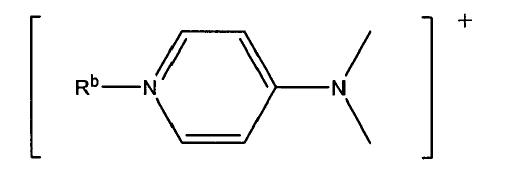 wherein Bas, Z and R^{b} are as defined above.

It is particularly preferred that Cat⁺ is a heterocyclic ring structure and Bas is a sterically hindered amino group, for example:

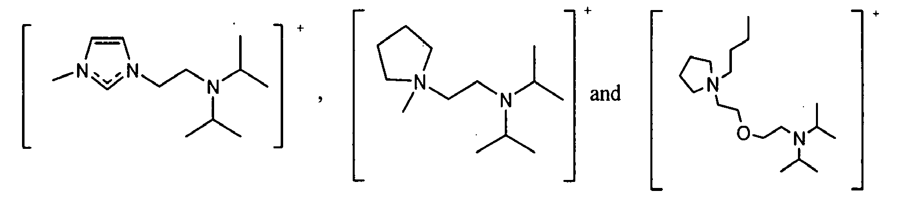

The heterocyclic Cat⁺ moiety may be obtained by alkylation, protonation and/or acylation of a precursor selected from imidazoles, pyridines, pyrazoles, thiazoles, isothiazoles, azathiozoles, oxothiazoles, oxazines, oxazolines, oxazoboroles, dithiozoles, triazoles, selenozoles, oxaphospholes, pyrroles, boroles, furans, thiophenes, phospholes, pentazoles, indoles, indolines, oxazoles, isooxazoles, isotriazoles, tetrazoles, benzofurans, dibenzofurans, benzothiophenes, dibenzothiophenes, thiadiazoles, pyrimidines, pyrazines, pyridazines, piperazines, piperidines, morpholenes, pyrans, annolines, phthalzines, quinazolines, quinoxalines, quinolines, isoquinolines, thazines, oxazines, and azaannulenes.

It is also envisaged that the Cat⁺ moiety may be an acyclic hydrocarbyl moiety. Preferably, the acyclic hydrocarbyl moiety comprises a group selected from amino amidino, imino, guanidino, phosphino, arsino, stibino, alkoxyalkyl, alkylthio, alkylseleno and phosphinimino.

Where the Cat⁺ moiety is an acyclic hydrocarbyl moiety, [Cat⁺-Z-Bas] is preferably selected from:

[N(Z-Bas)(R^{b})(R^{c})(R^{d})]⁺ and [P(Z-Bas)(R^{b})(R^{c})(R^{d})]⁺

wherein Bas, Z, R^{b}, R^{c}, and R^{d} are as defined above. It is particularly preferred that R^{b}, R^{c}, and R^{d} are independently selected from methyl and ethyl. Examples of preferred [Cat⁺-Z-Bas] of this class include: 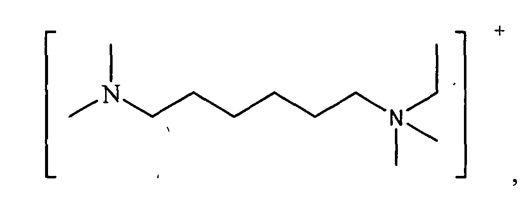 and 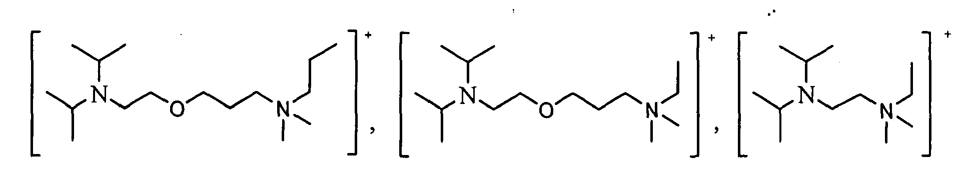 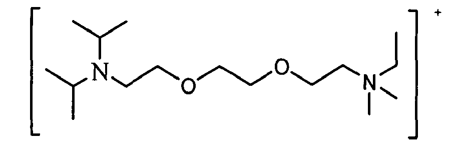 where Bas is the sterically hindered amino group, -N(CH(CH₃)₂)₂.
[Cat⁺-Z-Bas] may also be: 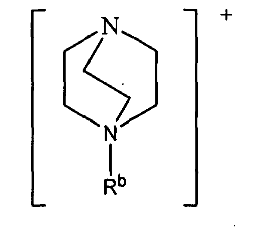 wherein R^{b} is as defined above.

Where the cation, C⁺, has a basic moiety, the anion, A⁻, may be any anion that forms a liquid salt with the cation at a temperature below 150°C, preferably below 100°C. Accordingly, the anion, A⁻, may be a conventional anion for an ionic liquid i.e. the anion does not have a basic moiety. Suitable conventional anions, A⁻, include hydroxide, alkoxide, phenoxide, dicyanamide, borate, phosphate, nitrate, sulfate, antimonate, phosphite, substituted and unsubstituted metalloborane, substituted and unsubstituted carboxylate and triflate; or mixtures thereof. Preferred conventional anions, A⁻, are selected from BF₄⁻, PF₆⁻, CF₃SO₃⁻ (triflate), CF₃COO⁻, SbF₆⁻, CuCl₂⁻, A₅F₆⁻, CF₃CH₂CH₂COO⁻, [(CF₃SO₂)₃C]⁻, CF₃(CF₂)₃SO₃⁻, bistrifluoromethanesulfonylamide [CF₃SO₂]₂N⁻, alkylsulfonate [RSO₃⁻], and metal inorganic anions.

The basic ionic liquid may also be of the reverse type where the anion, A⁻, has a basic moiety and the cation, C⁺ is any cation that forms a liquid salt with the anion at a temperature below 150°C, preferably below 100°C, and the use of such ionic liquids forms a preferred embodiment of the process of the invention. In these ionic liquids, the cation may be any conventional cation for an ionic liquid i.e. a cation that does not necessarily have a basic moiety.

Suitably an anion, A⁻, having a basic moiety is of general formula:

[An⁻-(Z-Bas)]

where An⁻ is an anionic moiety and Z and Bas are as defined above for the cation, C⁺. Preferably, An⁻ is a carboxylate or sulfonate group, CO₂⁻ or SO₃⁻. Bas may for example be an amino group of formula -N(R¹)(R²) as defined above, for example, -N(CH₃)₂, preferably a hindered amino group such as -N(CH(CH₃)₂)₂ or -N(C(CH₃)₃)₂. Preferably however Bas in an anion A⁻ is a heterocyclic ring system containing a basic nitrogen atom, for example a pyrrolidine, piperidine or morpholine ring, which may if desired be optionally substituted, for example by one or more C₁₋₄alkyl groups, and/or may if desired carry a keto (O=) group.

A convenient source of basic anions is via amino acids. Amino acids, for example having the formula:

R¹R²N-Z-CO₂H or Het-Z-CO₂H

alternatively represented in zwitterionic form as:

HR¹R²N⁺-Z-CO₂⁻ or Het-Z-CO₂⁻

in which each of R¹, R² and Z has the meaning given above, and Het represents a heterocyclic ring system containing a basic nitrogen atom, may be reacted with a suitable hydroxide salt to give a basic ionic liquid. The hydroxide may for example be a quaternary ammonium or phosphonium salt of the formula [YR¹R²R³R⁴]⁺OH⁻ where Y is a phosphorus or, preferably, nitrogen atom, giving an ionic liquid of the formula:

[R¹R²N-Z-CO₂]⁻ [YR¹R²R³R⁴]⁺ or [Het-Z-CO₂]⁻ [YR¹R²R³R⁴]⁺

Any suitable amino acid may be used. Simple amino acids of the general formula

R¹R²N-(CR^{x}R^{y})ₘ-CO₂H

in which each of R¹ and R² has the meaning given above (and preferably each of R¹ and R² independently represents a hydrogen atom, a methyl group or an ethyl group), each of R^{x} and R^{y} independently represents a hydrogen atom, a methyl group or an ethyl group, and m represents an integer from 1 to 6, may be used. Some of these are naturally occurring; naturally-occurring amino acids which may be used include alanine, arginine, asparagines, aspartic acid, cysteine, glutamic acid, glutamine, glycine, isoleucine, leucine, lysine, methionine, proline, serine, threonine, tryptophan, tyrosine, and valine, and analogues and derivatives of these acids such as sarcosine, 2-aminobutyric acid, 2-aminoisobutyric acid, canaline and creatine, may also be used. The use of amino acids based on nitrogen-containing heterocycles, for example, pyrrolidone-5-carboxylic acid, pipecoline, 1-piperidinepropionic acid and proline, may in some circumstances be preferred.

The sulfonic acid analogues of the above amino acids, i.e. those compounds in which the CO₂ group is replaced by an SO₃ group, may also be used. Such compounds include for example taurine and 2-(cyclohexylamino)ethanesulfonic acid.

Any convenient hydroxide salt may be used provided that the reaction product with the amino acid is an ionic liquid. The use of quaternary ammonium hydroxides is preferred. Tetralkylammonium hydroxides, for example tetrabutylammonium hydroxide, are for example suitable. Choline hydroxide, [(CH₃)₃NCH₂CH₂OH]⁺OH, has been used successfully, and its use is convenient for environmental and toxicity reasons.

It is also envisaged that both the cation and the anion of the ionic liquid may have a basic moiety. Thus for example the ionic liquid may be of general formula:

[Cat⁺-(Z-Bas)ₙ][An⁻-Z-Bas].

Basic ionic liquids containing a basic anion, are believed to be novel, and the invention therefore also provides these compounds *per se*.

The basic ionic liquid may comprise a mixture of one or more cations, C⁺. The basic ionic liquid may comprise a mixture of one or more anions, A⁻.

The basicity of the basic ionic liquid may be altered by adjusting the distance between the cationic (or anionic) moiety and the basic moiety. For example, where Z is a divalent alkylene group and Bas is an amino group, a separation of 2 methylene groups gives a weakly basic ionic liquid while a separation of at least 6 methylene groups gives a more strongly basic ionic liquid.

Preferably, the basic ionic liquid is immiscible with the oil. By immiscible with the oil is meant that the basic ionic liquid is soluble in the treated oil phase at a concentration of less than 50 ppm, preferably less than 30 ppm, more preferably less than 20 ppm, most preferably, less than 10 ppm, for example, less than 5 ppm. Thus, the solubility of the basic ionic liquid is tailored so that the basic ionic liquid is immiscible with the oil.

The solubility of the basic ionic liquid may also be tailored such that the basic ionic liquid is either insoluble or soluble in water. By insoluble in water is meant that the basic ionic liquid has a solubility in water of less than 50 ppm, preferably, less than 30 ppm, more preferably less than 20 ppm, most preferably, less than 10 ppm, for example, less than 5 ppm.

Suitably, the contacting step (a) of the process of the present invention is carried out at a temperature of from ambient to 150°C. Suitably, the contacting step (a) is carried out at a pressure of from 0.1 M Pa absolute to 10 M Pa absolute (1 bar absolute to 100 bar absolute).

Step (a) may be carried out by contacting the oil with the basic ionic liquid in a vessel wherein the resulting mixture is stirred using, for example, a mechanical stirrer, an ultrasonic stirrer, an electromagnetic stirrer or by bubbling an inert gas through the mixture. Suitably, the volume ratio of basic ionic liquid to oil in the extraction step is from 1:1 to 1:40, preferably, 1:4 to 1:20, for example 1:10 with the proviso that the amount of basic ionic liquid employed in contacting step (a) should be at least equivalent to the amount of organic acid in the oil. The mixing step may last from 1 minute to 60 minutes, preferably 2 to 30 minutes, more preferably, 5 to 20 minutes and most preferably, 8 to 15 minutes.

Where the basic ionic liquid is water soluble, and the oil to be treated using the process of the present invention has a high water content it may be necessary to dehydrate the oil prior to contacting the oil with the basic ionic liquid in step (a). The water may be separated from the oil in, for example, a separator or coalescer. Preferably, the concentration of water in the oil is less than 0.5 % volume of oil, for example, less than 0.25 % volume of oil.

Where the basic ionic liquid is insoluble in water, it is believed that any water present in the mixture (arising from the oil) may be beneficial in achieving the clean separation of the basic ionic liquid from the treated oil in step (b). Accordingly, it is not necessary to dehydrate the oil prior to step (a).

Step (b) may be carried out by gravity separation, (for example, in a settling unit) where the treated oil is generally the upper phase and the basic ionic liquid the lower phase in the settling unit. Where the basic ionic liquid is insoluble in water, the presence of the water will result in a 3 phase mixture where the treated oil is generally the upper phase, the water the middle phase and the basic ionic liquid containing the organic acids the lower phase in the settling unit. The phases may also be separated in step (b) using, for example, a decanter, a hydrocyclone, electrostatic coalescer or a centrifuge. Step (a) followed by step (b) may be repeated several times, preferably 2 to 6, for example 2 to 4 times, until the level of organic acids in the oil is reduced to an acceptable value.

Steps (a) and (b) may also be carried out together in a counter-current extraction column. The oil contaminated with organic acids (hereinafter "oil feed stream") is generally introduced at or near the bottom of the counter-current extraction column and the basic ionic liquid (hereinafter "basic ionic liquid feed stream") at or near the top of the counter-current extraction column. An oil phase which is reduced in organic acid content (hereinafter "product oil stream") is withdrawn from the top of the column and a basic ionic liquid extract phase containing the extracted organic acids (hereinafter "extract stream") from at or near the bottom thereof. Preferably, the counter-current extraction column has a sump region for collecting the basic ionic liquid extract phase. Preferably, the oil feed stream is introduced to the counter-current extraction column immediately above the sump region. More than one counter-current extraction column may be employed, for example 2-6, preferably 2-3 columns arranged in series. Preferably, the counter-current extraction column is packed with a structured packing material, for example, glass Raschig rings, thereby increasing the flow path for the oil and basic ionic liquid through the column. Alternatively, the counter-current extraction column may contain a plurality of trays.

Steps (a) and (b) may also be carried out together in a centrifugal contact separator, for example, a centrifugal contact separator as described in US 4,959,158, US 5,571,070, US 5,591,340, US 5,762,800, WO 99/12650, and WO 00/29120 which are herein incorporated by reference. Suitable centrifugal contact separators include those supplied by Costner Industries Nevada, Inc. The oil contaminated with organic acids and the basic ionic liquid may be introduced into an annular mixing zone of the centrifugal contact separator. Preferably, the oil contaminated with organic acids and the basic ionic liquid are introduced as separate feed streams into the annular mixing zone. The oil, and basic ionic liquid are rapidly mixed in the annular mixing zone such that at least a portion of the organic acids are extracted from the oil into the basic ionic liquid. The resulting mixture is then passed to a separation zone wherein a centrifugal force is applied to the mixture to produce a clean separation of an oil phase and a basic ionic liquid extract phase. Preferably, a plurality of centrifugal contact separators are used in series, preferably, 2-6, for example 2-3. Preferably, the oil feed stream is introduced into the first centrifugal contact separator in the series while the basic ionic liquid feed stream is introduced into the last centrifugal contact separator in the series such that oil of progressively decreasing organic acid content is passed from the first through to the last centrifugal contact separator in the series while basic ionic liquid of progressively increasing organic acid content is passed from the last through to the first centrifugal contact separator in the series. Thus, the basic ionic liquid extract phase is removed from the first centrifugal contact separator and the oil phase of reduced acidity from the last centrifugal contact separator in the series.

The oil phase of reduced acidity (product oil stream) which is isolated from step (b) may be used directly or may be further processed, for example, by fractional distillation. If necessary, any residual basic ionic liquid that is present in the treated oil may be recovered by passing the product oil stream through a silica column such that the residual basic ionic liquid is adsorbed onto the silica column. The adsorbed basic ionic liquid may then be washed off the silica column using a solvent for the basic ionic liquid and the basic ionic liquid may be recovered by driving off the solvent at reduced pressure. Alternatively, the oil may be removed from the residual ionic liquid by hot gas stripping using, for example, hot nitrogen gas.

The basic ionic liquid extract phase which is isolated from step (b) comprises basic ionic liquid and extracted organic acids. The basic ionic liquid is recovered from the organic acids in a regeneration step (c) and the regenerated basic ionic liquid may then be recycled to step (a).

Suitable regeneration methods include: (1) extraction of the organic acids into a solvent that is immiscible with the basic ionic liquid; (2) vaporization of the organic acids at a reduced pressure and at a temperature less than the decomposition temperature of the ionic liquid, preferably, a temperature less than 200°C; (3) reaction of the organic acids within the basic ionic liquid to form: (i) products that are insoluble in the basic ionic liquid, (ii) products that are more readily extracted into a solvent that is immiscible with the basic ionic liquid, or (iii) volatile products that are more readily separated from the basic ionic liquid; (4) gas stripping wherein a hot gas, for example steam or nitrogen is passed through the ionic liquid to volatilize the organic acids; (5) extraction of the organic acids with a supercritical fluid, for example, liquefied carbon dioxide; (6) membrane separation (polymer-based, ceramic, zeolite and liquid-liquid systems) where the membrane is selectively permeable to the organic acids; and combinations of these methods.

Preferably, the organic acids contained in the separated basic ionic liquid extract phase are reacted with a Group 1 and/or Group 2 metal hydroxide such that at least a portion of the organic acids, preferably, substantially all of the organic acids, are converted into Group 1 and/or Group 2 neutralization salts thereof within the basic ionic liquid. For example, the basic ionic liquid extract phase may be contacted with solid Group 1 and/or Group 2 metal hydroxide. Without wishing to be bound by any theory, it is believed that the neutralization salts formed by the reaction of the Brønsted acid and the Brønsted base may precipitate from the basic ionic liquid and may therefore be readily separated therefrom. Alternatively, where the basic ionic liquid is insoluble in a polar solvent, the neutralization salts may be extracted from the basic ionic liquid extract phase into the polar solvent. By insoluble in the polar solvent is meant that the basic ionic liquid has a solubility in the polar solvent of less than 50 ppm, preferably, less than 30 ppm, more preferably, less than 20 ppm, most preferably, less than 10 ppm, for example, less than 5 ppm. Suitable polar solvents include water and polar organic solvents such as C₁ to C₆ aliphatic alcohols, in particular, methanol or ethanol. Where the basic ionic liquid is insoluble in the polar solvent, it is preferred to contact the basic ionic liquid extract phase with a solution of the Group 1 and/or Group 2 metal hydroxide in the polar solvent thereby generating a basic ionic liquid phase of reduced organic acid content and a polar solvent extract phase containing the Group 1 and/or Group 2 metal neutralization salts. Where the polar solvent is a polar organic solvent, the volume ratio of polar organic solvent to basic ionic liquid is typically less than 1:1, preferably less than 0.5:1, more preferably, less than 0.25:1, for example, less than 0.1:1. The polar organic solvent may then be recovered by volatilization of the solvent at reduced pressure leaving behind a solid residue comprising the Group 1 and/or
Group 2 metal neutralization salts. Accordingly, it is preferred to contact the basic ionic liquid extract phase with the minimum amount of the solution of the Group I and/or Group 2 metal hydroxide in the polar organic solvent. Preferably, the polar solvent is water resulting in a waste water stream containing the Group 1 and/or Group 2 metal neutralization salts of the organic acid. Where the method of the present invention is employed offshore on a hydrocarbon production platform, the water is preferably seawater and the waste water stream may be disposed of by, for example, being injected into a porous subterranean formation (waste water disposal zone). Thus, higher amounts of water may be employed than polar organic solvent.

The Group 1 metal hydroxide may be selected from lithium hydroxide, sodium hydroxide, and potassium hydroxide, preferably, sodium hydroxide. Suitably, the Group IIA metal hydroxide is selected from barium hydroxide, magnesium hydroxide, and calcium hydroxide, preferably, calcium hydroxide. Mixtures of Group 1 and/or Group 2 metal hydroxides may be employed. However, it is preferred to employ a Group 2 metal salt or mixtures thereof owing to the risk of Group 2 metal salts of the organic acids forming soaps that can interfere with the separation of the polar solvent from the basic ionic liquid. Particularly preferred is calcium hydroxide.

It is envisaged that where the treated oil is a hydrocarbon that has been produced offshore from a porous hydrocarbon bearing formation, that the basic ionic liquid may be contacted with a brine, for example, seawater or a produced water, on a production platform where the pH of the seawater is adjusted using a base to a value of at least 8, preferably at least 10 during the contacting step. Seawater contains naturally occurring Group 1 and Group 2 metal ions in an amount sufficient to complex with the organic acids contained in the basic ionic liquid. Typically, the pH of the brine may be adjusted to a value in the range 10 to 12 so that the pH of the brine after neutralization of the organic acids is in the range 4 to 9. The pH of the seawater may be adjusted using a Group 1 and/or 2 metal hydroxide, for example, sodium hydroxide.

Where the basic ionic liquid is insoluble in the polar solvent, it is envisaged that the oil containing the organic acids, the basic ionic liquid and the solution of the Group 1 and/or 2 metal hydroxide in the polar solvent (preferably, water or methanol) may be mixed together in a stirred vessel followed by separation of an oil phase of reduced organic acid content, a polar solvent phase containing Group 1 and/or 2 neutralization salts of the organic acids and a basic ionic liquid phase. It is also envisaged that the oil containing the organic acids may be contacted with the basic ionic liquid and the solution of a Group 1 and/or Group 2 metal hydroxide in the polar solvent (preferably, water or methanol) in a counter-current extraction column. For example, the oil containing the organic acids and an aqueous solution of the Group 1 and/or 2 metal hydroxide may be introduced at or near the bottom of the column and the basic ionic liquid at or near the top thereof. Thus, oil having a reduced organic acid content is removed from at or near the top of the column, basic ionic liquid from at or near the bottom of the column and an aqueous solution of Group 1 and/or 2 metal neutralization salts of the organic acids from an intermediate position. Alternatively, a solution of the Group 1 and/or 2 metal hydroxide in methanol may be introduced into the column, at or near the bottom thereof and, depending upon the density of the oil, a solution of the Group 1 and/or Group 2 metal neutralization salts of the organic acids in methanol may be removed from either at or near the top of the column with the oil of reduced organic acid content being removed from an intermediate position or the oil of reduced organic acid content may be removed from at or near the top of the column and the solution of the Group 1 and/or Group 2 metal neutralization salts of the organic acids from an intermediate position.

It is also envisaged that the basic ionic liquid containing the organic acids may be contacted with a C₁ to C₆ aliphatic alcohol in the presence of an esterification catalyst (for example, a heterogeneous or homogeneous esterification catalyst) under conditions effective to convert at least a portion of the organic acids into the corresponding esters thereof. These ester derivatives are more volatile than the organic acids and hence are more readily separated from the basic ionic liquid, for example, by vaporisation at reduced pressure and at a temperature of less than 150°C.

Contacting step (a) and separation step (b) may also be carried out together by passing the oil through a column packed with a supported basic ionic liquid. Methods for supporting an ionic liquid on a support material are well known in the art. Typically, the basic ionic liquid may be physiosorbed or chemisorbed on the support material, preferably, chemisorbed. Suitable support materials include silica. Thus, the oil containing the organic acids may be passed through a column containing the supported basic ionic liquid. The organic acids will become associated with the supported basic ionic liquid and oil having a reduced organic acid content will be removed from the column. The supported basic ionic liquid may be regenerated by passing a solution of a Group 1 and/or Group 2 metal hydroxide in a polar solvent through the column such that the organic acids are converted into their corresponding neutralization salts and are washed off the column by the polar solvent. Suitably, the polar solvent is water or a C₁ to C₆ aliphatic alcohol or mixtures thereof. Preferably, the polar organic solvent is water, methanol or ethanol. Where the basic ionic liquid is physiosorbed onto the silica column, the basic ionic liquid should be insoluble in the polar solvent so that the basic ionic liquid is not stripped from the column. Where the polar solvent is water, the resulting waste water stream may be disposed of, for example, by being injected into a porous subterranean formation (waste water disposal zone). Where the polar organic solvent is methanol or ethanol, the solvent may be driven off from the neutralization salts at reduced pressure.

The process of the present invention may be carried out on an offshore platform, in a refinery, or whilst the oil is being transported, for example, in a tanker at sea.

The present invention will now be described with reference to the following Figures which represent schematic diagrams of two embodiments of the process of the present invention.

In Figure 1, an oil contaminated with organic acids (1) is fed to a first counter-current extraction column (2) at a point above a sump region (3) and comes into liquid-liquid contact with a basic ionic liquid (4) which is fed to the upper part of the column (2). The basic ionic liquid and the oil pass in a counter-current fashion through the first counter-current column (2) such that the organic acids which contaminate the oil are extracted into the basic ionic liquid. A basic ionic liquid stream (5) containing the extracted organic acids is withdrawn from the sump region (3). An oil stream of reduced organic acid content (6) is withdrawn from the top of the first counter-current column (2). The basic ionic liquid stream (5) containing the extracted organic acids is then fed to the upper part of a second counter-current column (7) while a solution of sodium hydroxide in methanol (8) is fed to the second counter-current column (7) at a point above a sump region (9). A solution of the sodium neutralization salts of the organic acids in methanol (10) is withdrawn from the top of the second counter-current column (7) while a basic ionic liquid stream of reduced organic acid content (11) is withdrawn from the sump region (8). The methanol may be recovered from the sodium neutralization salts by vaporization of the methanol at low pressure (not shown).

Figure 2 illustrates a further embodiment of the process of the present invention in which oil contaminated with organic acids (20) and the basic ionic liquid (21), are fed to a stirred tank (22) such that the organic acids which contaminate the oil are extracted into the basic ionic liquid. The mixture of the oil and basic ionic liquid is withdrawn from the stirred tank via line (23) and is transferred to a separator tank (24) where the mixture separates into an upper oil phase of reduced organic acid content (25) and a lower basic ionic liquid phase containing extracted organic acids (26). If the oil has a high water content, three phases may separate with the middle layer comprising water. If necessary, water may be added to the mixture in the stirred tank (22) to assist in separation of the basic ionic liquid from the oil in the separator tank (24). The oil phase of reduced organic acid content is withdrawn from the separator tank (24) via line (27). The basic ionic liquid phase containing the extracted organic acids is withdrawn from the separator tank (23) and is transferred, via a line (28), to a counter-current column (not shown) where the organic acids are separated from the basic ionic liquid, by contacting the basic ionic liquid extract phase with a solution of sodium hydroxide in methanol, as described above.

The process of the present invention will now be illustrated by the following

### Examples.

### Synthesis A: Synthesis of the basic ionic liquid {6-(dimethylamino)hexyl}(ethyl)dimethylammonium bis{(trifluoromethyl) sulfonyl} amide

The basic ionic liquid {6-(dimethylamino)hexyl}(ethyl)dimethylammonium *bis*{(trifluoromethyl) sulfonyl} amide was prepared in two steps, the first step being the synthesis of the halide and the second step a metathesis reaction to form the basic ionic liquid.

### Step (a) - Synthesis of the halide 1- dimethylamino-6-ethyldimethylammoniumhexane iodide

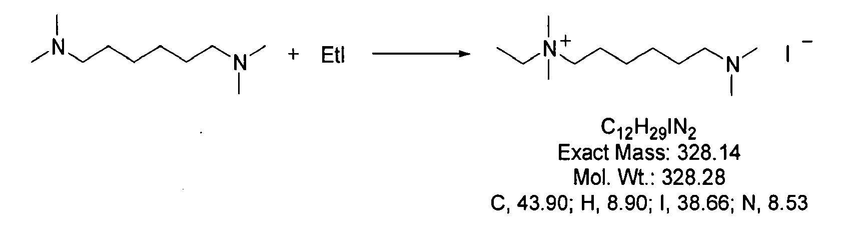

*N,N,N,N*-tetramethyl-hexane-1,6-diamine (17.89g, 103.81mmol) was dissolved in diethylether (400 ml) and iodoethane (16.19g, 103.81mmol) was added dropwise to this solution. The resulting solution was stirred at room temperature for 2 days, and the formation of a white precipitate was observed. The precipitate was separated by filtration, washed with ether and dried under high vacuum. The precipitate was suspended in 200ml of trichloromethane and filtered again. The filtrate contained the desired monoalkylated product. The solvent was removed *in vacuo*, leaving behind a white powder. (8.94g, 27.23mmol, 26% yield). The white powder was determined by NMR to be 1-dimethylamino-6-ethyldimethylammoniumhexane iodide.

### Step (b) - Metathesis

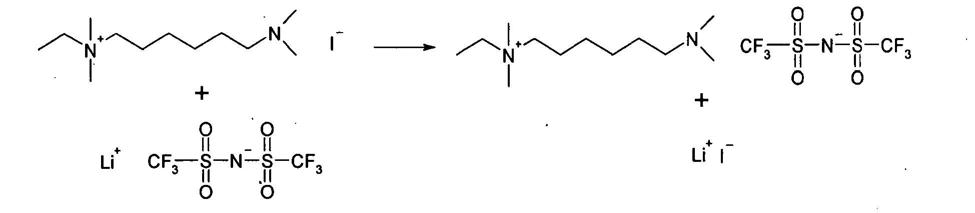

The halide formed in Step (a) (1- dimethylamino-6-ethyldimethylammoniumhexane iodide) was mixed with an equimolar quantity of Lithium *bis*{(trifluoromethyl)sulfonyl} amide in 200ml of water. The solution was stirred at room temperature for 1 day, whereupon the formation of a separate lower phase was observed. This lower phase was determined by NMR to be the desired basic ionic liquid {6-(dimethylamino)hexyl}(ethyl)dimethylammonium *bis*{(trifluoromethyl) sulfonyl} amide. The ionic liquid product was purified by dissolving the ionic liquid in 200ml of dichloromethane and extracting the solution of the ionic liquid three successive times with 100ml quantities of distilled water. This purification was designed to remove any residual LiCl remaining in the ionic liquid product. Finally, the dichloromethane solvent and any residual water was removed by vacuum distillation.
The basic ionic liquid was analyzed by NMR spectroscopy:
¹H-NMR (300 MHz, *d₃-trichloromethane*): δ = 1.20-1.46 (m, 9H, CH₂ and N⁺-CH₂*CH₃*);
1.59-1.75 (m, 2H, N⁺-CH₂*CH₂*); 2.12 (s, 6H, N-CH₃); 2.16 (t, *J* = 7.3 Hz, 2H, N-CH₂);
3.27 (s, 6H, N⁺-CH₃); 3.39-3.52 (m, 2H, N⁺-*CH₂*CH₃), 3.61 (q, *J=* 7.2 Hz, 2H, N⁺-*CH₂*CH₂).
¹³C-NMR (75 MHz) *d₃-trichloromethane*): δ = 9.19 (Et-CH₃); 22.71, 26.01, 26.90, 27.37
(CH₂); 45.46 (N-CH₃); 50.95 (N-CH₂); 59.34 (N⁺-CH₃); 59.78 (N⁺-CH₂CH₃); 63.71 (N⁺-CH₂CH₂).

### Example 1

A model hydrocarbon mixture was prepared by mixing 132.45g of hexane with 6.62g of cyclohexane carboxylic acid, which gives a Total Acid Number (TAN) of 20.87mg/g. The total acid number was calculated and the value verified through analysis with an autotitration apparatus using a standard procedure for the determination of TAN values of oil samples (as outlined in "standard test method for Acid Number of Petroleum Products by Potentiometric Titration", ASTM 0664-04). In a small vial, 7g of the model hydrocarbon, was mixed with 1g of the basic ionic liquid {6-(dimethylamino)hexyl}(ethyl)dimethylammonium *bis*{(trifluoromethyl)sulfonyl}amide (prepared as described above) and the two phase mixture shaken by hand at room temperature for approximately 2 minutes. The small vial also contained a small magnetic flea, to break interfacial tension between the two components. After mixing, the two phases were centrifuged at 3000 rpm for 20 minutes, after which the ionic liquid had formed a lower layer. The upper hydrocarbon layer was then sampled (1g), and the sample was then used for TAN analysis. The TAN analysis revealed that the TAN value had decreased from 20.87mg/g to 13.12mg/g.

### Comparative Example 1

In a small vial containing a magnetic flea, 7g of the model hydrocarbon mixture, was mixed with 1g of a non-basic ionic liquid 1-methyl-1*H*-imidazol-3-ium *bis*{(trifluoromethyl)sulfonyl}amide and the two phase mixture was shaken by hand at room temperature for approximately 2 minutes. The mixture was then centrifuged at 3000 rpm for 20 minutes after which the ionic liquid had formed a lower layer. The upper hydrocarbon layer was then sampled (1g), and the sample was then used for TAN analysis. The TAN analysis revealed that the TAN value had decreased from 20.87mg/g to 20.12mg/g. This decrease is not significant in comparison to the extraction with basic ionic liquid of Example 1 and therefore confirms that an economic ionic liquid extraction processes requires a basic site incorporated into the ionic liquid.

### Example 2

To further demonstrate the principle of the deacidification method of the present invention, a model oil more similar in nature to that of a crude oil was created by spiking 145.8g of kerosene with 1.85g of naphthenic acid, to form a kerosene/naphthenic acid mixture with a TAN value of 2.82 mg/g. In a small vial containing a magnetic flea, 7g of the kerosene/naphthenic acid, was mixed with 1g of the same basic ionic liquid as described in Example 1 and the vial was shaken by hand at room temperature for approximately 2 minutes. The mixture was then centrifuged at 3000 rpm for 20 minutes, after which the upper hydrocarbon layer was sampled (5g), and the sample was used for TAN analysis. The TAN analysis revealed that the TAN value had decreased from 2.82mg/g to 0.52mg/g.

### Example 3

A crude oil sample from the Harding oil field in the North Sea was found to have a TAN of 2.86 mg/g. In a small vial, 7g of the Harding crude oil, was mixed with 1g of the basic ionic liquid {6-(dimethylamino)hexyl}(ethyl)dimethylammonium *bis*{(trifluoromethyl)sulfonyl}amide and the two phase mixture was shaken by hand at room temperature for approximately 2 minutes. The small vial also contained a small magnetic flea to help break interfacial tension between the two components. After mixing, the two phases were centrifuged at 3000 rpm for 20 minutes, after which the ionic liquid had formed a lower layer. The upper hydrocarbon layer was then sampled (5g), and the sample was used for TAN analysis. The TAN analysis revealed that the TAN value had decreased from 2.86mg/g to 1.92mg/g.

### Comparative Example 2

In a small vial, 7g of the same crude oil as described in Example 3, was mixed with 1g of the non-basic ionic liquid 1-butyl-3-methyl-1*H*-imidazolium *bis*{(trifluoromethyl)sulfonyl}amide ionic and the mixture was shaken by hand at room temperature for approximately 2 minutes. The small vial also contained a small magnetic flea, to break interfacial tension between the two components. After mixing, the two phases were centrifuged at 3000 rpm for 20 minutes, after which the ionic liquid had formed a lower layer. The upper hydrocarbon layer was then sampled (5g), and the sample was used for TAN analysis. The TAN analysis revealed that the TAN value had decreased from 2.86mg/g to 2.84mg/g. As this decrease is minor in comparison to Example 3, this comparative example provides further evidence that a successful commercial deacidification process requires the use of an ionic liquid containing a basic site.

### Example 4

In a small vial, 7g of the same crude oil as described in Example 3, was mixed with 2g of the same basic ionic liquid as described in Example 1 and the mixture shaken by hand at room temperature for approximately 2 minutes. The small vial also contained a small magnetic flea, to help break interfacial tension between the two components. After mixing, the two phases were centrifuged at 3000 rpm for 20 minutes, after which the ionic liquid had formed a lower layer. The upper hydrocarbon layer was then sampled (5g), and the sample was used for TAN analysis. The TAN analysis revealed that the TAN value had decreased from 2.86mg/g to 1.88mg/g.

### Example 5

In a small vial, 7g of the same crude oil as described in Example 3, was mixed with 4g of the same basic ionic liquid as described in Example 1 and the mixture was shaken by hand at room temperature for approximately 2 minutes. The small vial also contained a small magnetic flea, in order to help break interfacial tension between the two components. After mixing, the two phases were centrifuged at 3000 rpm for 20 minutes, after which the ionic liquid had formed a lower layer. The upper hydrocarbon layer was then sampled (5g), and the sample was used for TAN analysis. The TAN analysis revealed that the TAN value had decreased from 2.86mg/g to 1.84mg/g.

### Example 6

In a small vial, 14g of the same crude oil as described in Example 3, was mixed with 2g of the same basic ionic liquid as described in Example 1 and the mixture was shaken by hand at room temperature for approximately 2 minutes. The small vial also contained a small magnetic flea, in order to help break interfacial tension between the two components. After mixing, the two phases were centrifuged at 3000 rpm for 20 minutes, after which the ionic liquid had formed a lower layer. The upper hydrocarbon layer was then sampled (7g), which was then added to a fresh gram of the same basic ionic liquid as described in Example 1. As before, the resulting mixture was shaken by hand for approximately 2 minutes at room temperature, prior to being centrifuged at 3000 rpm for 20 minutes, whereby a lower ionic liquid layer was formed. The upper hydrocarbon layer was then sampled (5g), which was then used for TAN analysis. The TAN analysis revealed that the TAN value had decreased from 2.86mg/g to 1.56mg/g.

### Synthesis B: Synthesis of Choline Hydroxide

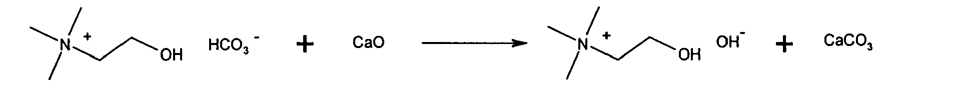

Aqueous choline bicarbonate solution, 75 % by weight (63.76g, 0.2896 mol choline bicarbonate) was diluted with ultra pure water (150 ml). Calcium oxide (16.29g, 0.2905 mol) was slowly added to cold ultra pure water (200 ml) forming a cloudy white suspension. The choline bicarbonate solution was slowly added to the calcium oxide suspension, with stirring, and allowed to stir for 12 hours. The resultant precipitate was filtered, washed with ultra pure water, and discarded. A representative sample of the colourless filtrate was then titrated against a primary acid, potassium acid phthalate, to determine hydroxide concentration. The above procedure produced about 350 ml of 0.66 molar choline hydroxide.

### Synthesis of Choline Prolinate (1)

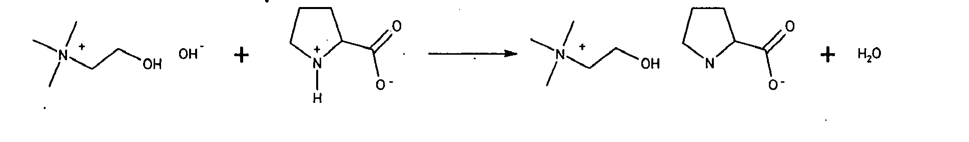

Proline (22.80g, 0.1980 mol) was dissolved in water (100 ml), and the solution slowly added to 0.66 M aqueous choline hydroxide (300.0 ml, 0.198 mol) and stirred for 12 hours. The resultant solution was transferred to a round bottomed flask, and the solvent (water) was removed *in vacuo* resulting in a colourless viscous, glassy liquid which may solidify under some conditions. The liquid was determined by NMR to be choline prolinate.

### Synthesis of Choline Prolinate (2)

Commercial choline hydroxide solution in methanol (45% by weight, 11.00g, 0.0408 mol) was added to a suspension of Proline (4.70g, 0.0408 mol) in 50 ml methanol, and the solution was stirred for 12 hours. The dark yellow-brown solution was treated with activated charcoal, filtered, and the filtrate was removed *in vacuo* resulting in a yellow viscous, glassy liquid. The liquid was determined by NMR to be choline prolinate.

### Example 7.

A crude oil sample from the Harding oil field in the North Sea was found to have a TAN of 2.86 mg/g. In a small vial, 7g of the Harding crude oil, was mixed with 1g of the basic anion ionic liquid choline prolinate and the two phase mixture was shaken by hand at room temperature for approximately 2 minutes. The small vial also contained a small magnetic flea to help break interfacial tension between the two components. After mixing, the two phases were centrifuged at 3000 rpm for 20 minutes, after which the ionic liquid had formed a lower layer. The upper hydrocarbon layer was then sampled (5g), and the sample was used for TAN analysis. The TAN analysis suggested complete removal of acids. IR analysis of Harding crude oil before contact with choline prolinate showed a distinctive carbonyl stretch at around 1700 cm⁻¹. IR analysis of Harding crude after contact with, and separation from, choline prolinate showed an absence of this peak.

## Claims

1. A process for deacidifying a crude oil and/or crude oil distillate containing organic acids comprising the steps of:
(a) contacting the crude oil and/or crude oil distillate containing organic acids with a basic ionic liquid having a melting point of below 150°C, and extracting at least a portion of the organic acids into the basic ionic liquid as an extract phase; and
(b) separating a crude oil and/or crude oil distillate phase which is reduced in acidity from the basic ionic liquid extract phase.

2. A process as claimed in claim 1, in which the material to be deacidified is crude oil or a crude oil distillate selected from liquefied petroleum gas, gasoline, gas oil, diesel, jet fuel, kerosene, home heating oil, and mixtures thereof.

3. A process as claimed in claim 2, in which the basic ionic liquid has a melting point of below 100°C.

4. A process as claimed in any one of the preceding claims, in which the cation present in the basic ionic liquid is represented by the formula:
Cat⁺-(Z-Bas)ₙ
wherein:
Cat⁺ is a positively charged moiety;
Bas is a basic moiety;
Z is a covalent bond joining Cat⁺ and Bas, or is a divalent linking group; and
n is an integer of from 1 to 3.

5. A process as claimed in claim 4, in which n is 1.

6. A process as claimed in either claim 4 or claim 5, in which Bas comprises a heterocyclic ring system containing a basic nitrogen atom.

7. A process as claimed in either claim 4 or claim 5, in which Bas represents a group of formula -N(R¹)(R²), -P(R¹)(R²), -SR³, or -OR³, in which R¹ and R² are independently selected from hydrogen, alkyl, cycloalkyl, aryl and substituted aryl, or, in the case of an -N(R¹)(R²) group, R¹ and R² together with the interjacent nitrogen atom form part of a heterocyclic ring, and R³ is selected from alkyl, cycloalkyl, aryl and substituted aryl.

8. A process as claimed in claim 7, in which R¹, R² and R³ are selected from methyl, ethyl, n-propyl, isopropyl, n-butyl, iso-butyl, tert-butyl, n-pentyl, n-hexyl, cyclohexyl, benzyl and phenyl, or, in the case of an -N(R¹)(R²) group, R¹ and R² together represent a tetramethylene or pentamethylene group optionally substituted by one or more C₁₋₄ alkyl groups.

9. A process as claimed in claim 8, in which Bas represents -N(CH(CH₃)₂)₂ or -N(C(CH₃)₃)₂.

10. A process as claimed in any one of claims 4 to 9, in which Z is a divalent organic radical having from 1 to 18 carbon atoms.

11. A process as claimed in claim 10, in which Z represents a divalent alkylene radical selected from:
-(CH₂-CH₂)-, (CH₂-CH₂-CH₂)-, -(CH₂-CH₂-CH₂-CH₂)-, -(CH₂-CH₂-CH₂-CH₂-CH₂)-, -
(CH₂-CH₂-CH₂-CH₂-CH₂-CH₂)-, -(CH₂-CH(CH₃))-, and -(CH₂-CH(CH₃)-CH₂-CH(CH₃))-;
(b) a divalent alkyleneoxyalkylene radical selected from:
- (CH₂₋CH₂-O-CH₂-CH₂)-, -(CH₂-CH₂-O-CH₂-CH₂-CH₂)-, and -(CH₂₋CH(CH₃)-OCH₂-CH(CH₃))-;
(c) a divalent polyoxyethylene radical selected from:
-(CH₂CH₂O)ₙ- where n is an integer in the range 1 to 9 or -(CH₂CH(CH₃)O)ₘ- where m is an integer in the range 1 to 6; or
(d) a divalent alkylenearylene or an alkylenearylenealkylene radical selected from:
-(CH₂-C₆H₄)-, and -(CH₂-C₆H₄-CH₂)-.

12. A process as claimed in any one of claims 4 to 11, in which Cat⁺ represents a heterocyclic ring structure selected from imidazolium, pyridinium, pyrazolium, thiazolium, isothiazolinium, azathiozolium, oxothiazolium, oxazinium, oxazolium, oxaborolium, dithiozolium, trizdium, selenozolium, oxaphopholium, pyrollium, borolium, furanium, thiophenium, phospholium, pentazolium, indolium, indolinium, oxazolium, isooxazolium, isotriazolium, tetrazolium, benzofuranium, diborzofuranium, benzothiophenium, dibenzothiophenium, thiadiazolium, pyrimidinium, pyrazinium, pyridazinium, piperazinium, piperidinium, morpholenium, pyranium, annolinium, phthalzinium, quinazolinium, quinaxalinium, quinolinium, isoquinolinium, thazinium, oxazinium and azaannulenium.

13. A process as claimed in any one of claims 5 to 10, in which Cat⁺-Z-Bas represents: 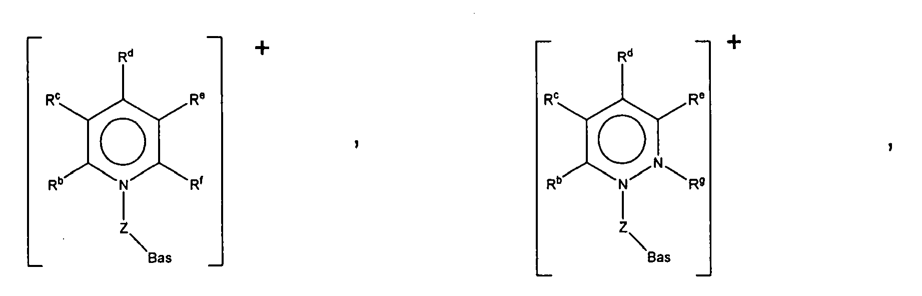 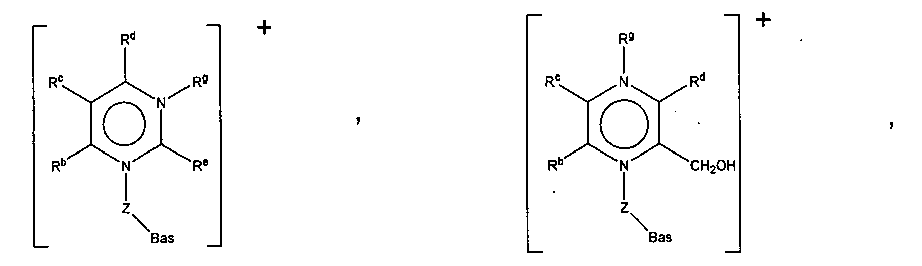 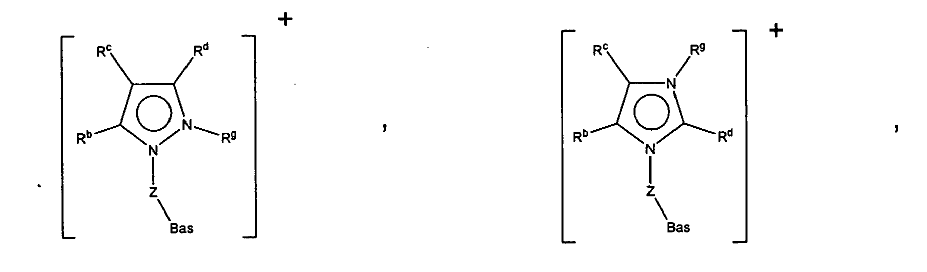 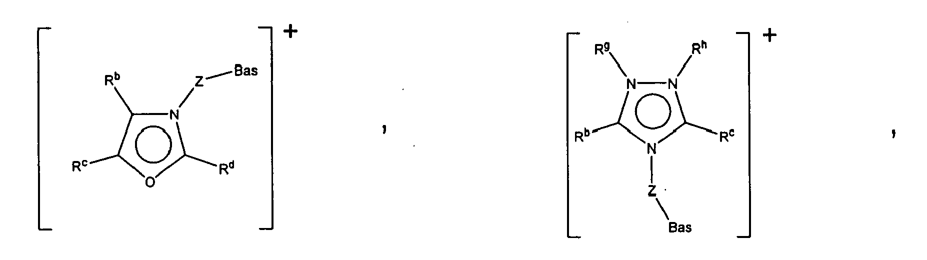 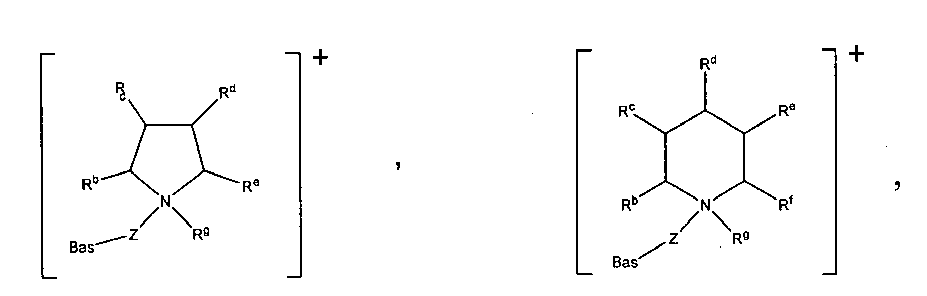 or 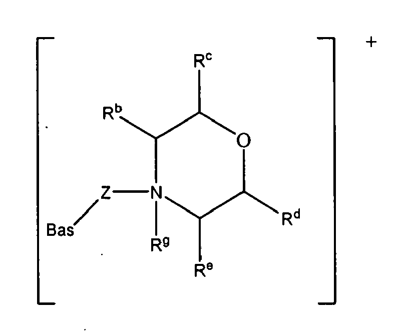 wherein Bas and Z are as defined as in any one of claims 4 to 10; and R^{b}, R^{c}, R^{d}, R^{e}, R^{f}, R^{g} and R^{h} are independently selected from hydrogen, a C₁ to C₄₀ alkyl group, a C₃ to C₈ cycloalkyl group, or a C₆ to C₁₀ aryl group, wherein said alkyl, cycloalkyl or aryl groups are unsubstituted or may be substituted by one to three groups selected from: C₁ to C₆ alkoxy, C₆ to C₁₀ aryl, CN, OH, NO₂, C₇ to C₃₀ aralkyl and C₇ to C₃₀ alkaryl, or any two of R^{b}, R^{c}, R^{d}, R^{e} and R^{f} attached to adjacent carbon atoms on the ring structure form a methylene chain -(CH₂)ₚ- wherein p is an integer from 3 to 5.

14. A process as claimed in claim 5, in which Cat⁺ -Z-Bas is selected from:

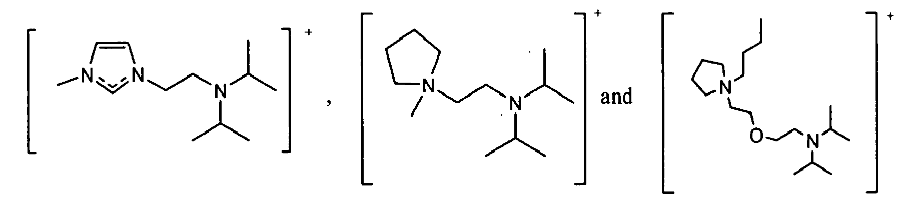

15. A process as claimed in claim 5, where Cat⁺-Z-Bas is selected from:
[N(Z-Bas)(R^{b})(R^{c})(R^{d})]⁺ and [P(Z-Bas)(R^{b})(R^{c})(R^{d})]⁺
wherein Bas and Z are as defined in any one of claims 4 to 10 and each of R^{b}, R^{c}, and R^{d} are independently selected from methyl and ethyl.

16. A process as claimed in claim 5, in which Cat⁺-Z-Bas is selected from: 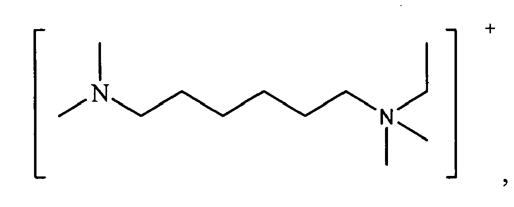 and

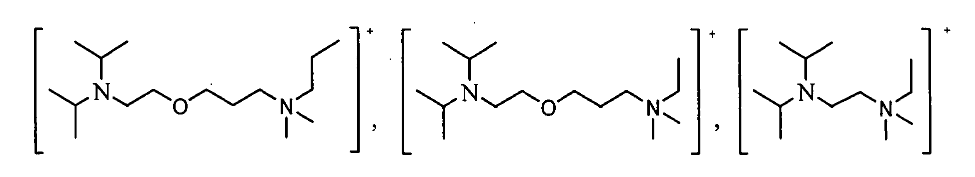

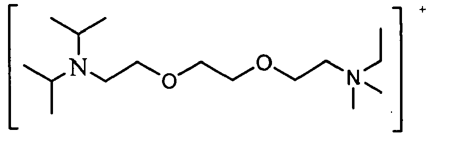

17. A process as claimed in any one of claims 1 to 3, in which the basic ionic liquid comprises an anion having a basic moiety.

18. A process as claimed in claim 17, in which the anion has the general formula:
An⁻-(Z-Bas)
where An⁻ is an anionic moiety and Z and Bas are as defined in any one of claims 4 to 11.

19. A process as claimed in claim 18, in which An⁻ is a CO₂⁻ or SO₃⁻ group.

20. A process as claimed in either claim 18 or claim 19, in which Bas represents a heterocyclic ring system containing a basic nitrogen atom.

21. A process as claimed in claim 20, in which Bas represents a pyrrolidine, piperidine or morpholine ring, which may if desired be optionally substituted by one or more C₁₋₄alkyl groups, and/or may if desired carry a keto (O=) group.

22. A process as claimed in claim 17, in which the basic ionic liquid is derived from an acid having the formula:
R¹R²N-Z-CO₂H or Het-Z-CO₂H
in which Z represents a covalent bond or a divalent linking group, and each of R¹ and R² are independently selected from hydrogen, alkyl, cycloalkyl, aryl and substituted aryl, or, together with the interjacent nitrogen atom, form part of a heterocyclic ring; and Het represents a heterocyclic ring system containing a basic nitrogen atom.

23. A process as claimed in claim 17, in which the basic ionic liquid is derived from a coumpound selected from alanine, arginine, asparagines, aspartic acid, cysteine, glutamic acid, glutamine, glycine, isoleucine, leucine, lysine, methionine, proline, serine, threonine, tryptophan, tyrosine, valine, sarcosine, 2-aminobutyric acid, 2-aminoisobutyric acid, canaline, creatine, pyrrolidone-5-carboxylic acid, pipecoline, 1-piperidinepropionic acid, proline, taurine and 2-(cyclohexylamino)ethanesulfonic acid.

24. A process as claimed in any one of claims 17 to 23, in which the cation present in the basic ionic liquid is as defined in any one of claims 4 to 16, or is a quaternary ammonium or quaternary phosphonium ion.

25. A process as claimed in any one of the preceding claims, in which the basic ionic liquid is soluble in the treated oil phase at a concentration of less than 50 ppm.

26. A process as claimed in any one of the preceding claims, in which the volume ratio of basic ionic liquid to oil in the extraction step is from 1:1 to 1:40, with the proviso that the amount of basic ionic liquid employed in contacting step (a) should be at least equivalent to the amount of organic acid in the oil.

27. A process as claimed in any one of the preceding claims, in which following step (b), the basic ionic liquid is recovered from the organic acids in a regeneration step (c) following which the regenerated basic ionic liquid is recycled to step (a).

28. A process as claimed in any one of the preceding claims, in which in step (c) the organic acids contained in the separated basic ionic liquid extract phase are reacted with a Group 1 and/or Group 2 metal hydroxide such that at least a portion of the organic acids are converted into Group 1 and/or Group 2 neutralization salts thereof within the basic ionic liquid.

29. A basic ionic liquid which comprises an anion having a basic moiety.

30. A basic ionic liquid as claimed in claim 29, and as defined in any one of claims 18 to 24.
